# EUROPEAN PATENT SPECIFICATION

(11) **EP 2 842 433 B1**
(45) Date of publication and mention of the grant of the patent: **06.06.2018**
(21) Application number: 13782047.8
(22) Date of filing: 26.04.2013
(51) Int. Cl.: A61K 36/062, A61K 36/07, A61K 36/53, A61P 37/00, A23L 31/00, A23L 33/105

(54) **FOOD FOR NUTRITIONAL THERAPY OF AIDS**
NAHRUNGSMITTEL ZUR ERNÄHRUNGSTHERAPIE VON AIDS
ALIMENT POUR LA THÉRAPIE NUTRITIONNELLE POUR LE SIDA

(30) Priority: 26.04.2012 CN 201210126929
(43) Date of publication of application: 04.03.2015
(73) Proprietor: Peng, Du, Jiangyin, Jiangsu 214408 (CN); Si, Jianjiang, Jiangyin, Jiangsu 214408 (CN)
(72) Inventor: Peng, Du, Jiangyin, Jiangsu 214408 (CN); Si, Jianjiang, Jiangyin, Jiangsu 214408 (CN)
(74) Representative: Grünecker Patent- und Rechtsanwälte PartG mbB
(86) International application number: PCT/CN2013/074756
(87) International publication number: WO 2013/159730

(56) References cited:
- CN-A- 1 098 244
- CN-A- 1 126 092
- CN-A- 1 363 371
- CN-A- 1 416 741
- CN-A- 1 742 888
- CN-A- 1 995 323
- CN-A- 102 133 283
- CN-A- 102 342 961
- DATABASE WPI Week 199902 Thomson Scientific, London, GB; AN 1999-018309 XP002744794, & JP H10 287583 A (ITO H) 27 October 1998 (1998-10-27)
- LI MIAO ET AL: "Purification and Characterization of a Laccase from the Edible Wild Mushroom Tricholoma mongolicum", JOURNAL OF MICROBIOLOGY AND BIOTECHNOLOGY, vol. 20, no. 7, July 2010 (2010-07), pages 1069-1076, XP002744795,

## Description

### TECHNICAL FIELD

The present invention relates to functional foods, especially to a food for the nutritional therapy of Acquired Immune Deficiency Syndrome (AIDS).

### BACKGROUND

Acquired Immune Deficiency Syndrome (AIDS) is a syndrome wherein a human is infected by Human Immunodeficiency Virus (HIV), resulting in immunodeficiency and the simultaneous occurrence of a series of opportunistic infections and tumors. In severe cases, death will be caused. Currently, the mortality rate of AIDS is as high as 100%, and AIDS has been classified as category B infectious disease - indeed one of the infectious diseases monitored by leading health and quarantine organizations. AIDS is known as a "super fatal disease" and has become public health problem which severely threatens human health worldwide.

Malnutrition further hinders physiological function owing to the damage it causes to the immune system, which reduces AIDS patients' quality of life and daily activity, shortens their survival time and negatively influences the efficacy of AIDS medicines. Because large numbers of erythrocytes are destroyed, AIDS patients commonly suffer from what is known in Traditional Chinese Medicine (hereafter TCM) as "fear of chill". Due to worsening organism function, the AIDS patients' ability to absorb nutrient substances also deteriorates, which results in the aggravation of erythrocyte damage, leading to a vicious cycle and the acceleration of the course of the disease. Thus, ensuring the supply and absorbance of nutrients is most crucial for AIDS patients.

Nutritional support therapy is intended to prevent and correct cacotrophy which has arisen, or may arise, during the course of the disease and the therapy of the patient. Nutrition support therapy includes enteral nutrition therapy and parenteral nutrition therapy. Enteral nutrition (EN) provides the nutrient substance required by the body via mouth (i.e. a feeding tube). Parenteral nutrition (PN) provides complete and sufficient nutrition intravenously in order to meet the body's requirements. When all nutrient substances are offered to fasting patients intravenously, this is known as total parenteral nutrition (TPN).

AIDS nutrition support therapy satisfies the nutritional needs of AIDS patients, improves patients' nutrition, enhances tolerance to therapy, promotes recovery from and prevention of AIDS, cures cacotrophy, strengthens the body's defense capacity, reduces complications, controls opportunistic infections, lowers mortality rates, allows AIDS patients to more easily receive treatments, slows the development of disease, increases AIDS patients' quality of life and extends their lifespan.
With the decrease of immunity in patients with AIDS, complications are also more likely, and the survival rate for patients in final stage of the syndrome is very low. Although there is no effective cure so far, attention to the patient's general health means that quality of life can be enhanced and disease progression slowed, which is good news for AIDS patients.

### SUMMARY

In view of the demand for therapies in the above-mentioned field versus their relative scarcity, this invention aims to provide a food for the nutritional therapy of AIDS. Said food is made from TCM materials or food materials. It can alleviate the symptom of "fear of chill" caused by excessive damage to erythrocytes, and can enhance immunity effectively without toxicity.

A food for the nutritional therapy of AIDS is prepared by fermenting the hyphae of a maternal strain of the common morel (*Morchella spp.*) and the hyphae of a maternal strain of the matsutake mushroom (*Tricholoma matsutake)* in a TCM liquid medium, said TCM liquid medium being an aqueous extract of the raw material of Purple Pearls beautyberry (*Callicarpa bodinieri).*

The weight ratio between the original raw material and the final aqueous extract of the raw material is 4∼5%.
Said TCM liquid medium also comprises adjuvants. The components of said adjuvants (by weight ratio) are as follows: 1 part pulse flour, 1 part corn flour, 2 parts white sugar, 0.1 part dipotassium hydrogen phosphate, 0.1 part potassium dihydrogen phosphate, 0.05 parts magnesium sulfate.
Said aqueous extract is extracted from the root of the Purple Pearls beautyberry (*Callicarpa bodinieri*).

The form of the food is granules, soup or powder.

The application of the food, wherein the application refers to the food as a component, involves its addition into food prepared for AIDS patients.

The preparation method of said food comprises the following steps:
(1) preparing aqueous extract of the raw material of Purple Pearls beautyberry (*Callicarpa bodinieri*)
(2) Diluting the aqueous extract by adding 20∼25 times its volume in water to obtain the TCM liquid medium;
(3) Injecting maternal strains of the common morel (*Morchella spp.)* and the matsutake mushroom (*Tricholoma matsutake)* into said TCM liquid medium and conducting fermentation.

In step (1), the weight ratio between the original raw material and the final aqueous extract of the raw material is 4∼5%.

In step (2) (adding the adjuvants into the aqueous extract, wherein the added adjuvants have 4∼5 times of weight of the raw material, and then conducting elixation), the constituents of said adjuvants (by weight ratio) are as follows: 1 part pulse flour, 1 part corn flour, 2 parts white sugar, 0.1 part dipotassium hydrogen phosphate, 0.1 part potassium dihydrogen phosphate, 0.05 parts magnesium sulfate.

Said fermentation comprises: inactivation by steaming the preparation with water under ordinary pressure, cooling to 26±2°C, and fermenting for 60∼144 hours.
Said aqueous extract is extracted from the root of the Purple Pearls beautyberry (*Callicarpa bodinieri*).
In said food of this invention for nutritional therapy, the nutrients in Purple Pearls beautyberry (*Callicarpa bodinieri*) and edible fungi combine to strengthen the physical function of the whole body and each individual organ of the AIDS patient, as well as to relieve the symptom of "fear of chill" by protecting erythrocytes and enhancing the regenerative capacity of erythrocytes in AIDS patients. Clinical tests prove that the food can be effective in improving survival quality, increasing body weight, strengthening immunity, promoting physical activity and relieving pain.

The root, stem, leaf, flower and/or seed of the Purple Pearls beautyberry (*Callicarpa bodinieri*) can be used as medicine. According to records in medical prior art, the Purple Pearls beautyberry (*Callicarpa bodinieri*) has the pharmaceutical effects of lowering fever, detoxication, blood-cooling, staunching bleeding, reducing inflammation, and promoting tissue regeneration, and also has anti-tumor effects, among other applications. Different parts of the Purple Pearls beautyberry (*Callicarpa bodinieri*) can cure different diseases with significant effects. The aqueous extract of the root of the Purple Pearls beautyberry (*Callicarpa bodinieri*) is used to cure ocular infection, fever, thirst, dysentery, relieving itching; the leaf is used for curing spitting blood, hemoptysis, hematochezia, uterine bleeding, and traumatic hemorrhage. The leaf and young stem of the Purple Pearls beautyberry (*Callicarpa bodinieri*) can be collected in spring, summer, and autumn, and used directly or dried in the sun and ground to a powder. The root can be collected year-round, sliced and dried in the sun.
Said edible fungi of this invention comprise the common morel (*Morchella spp.)* and the matsutake mushroom (*Tricholoma matsutake).* Maternal strains of the common morel (*Morchella spp.)* and that of the matsutake mushroom (*Tricholoma matsutake)* are mixed and fermented with an aqueous extract of the Purple Pearls beautyberry (*Callicarpa bodinieri*). By fermenting the strains, and combining the alimentotherapeutic effects of the two edible fungi and the pharmaceutical effects of the Purple Pearls beautyberry (*Callicarpa bodinieri*), the symptom of "fear of chill" and a series of complications due to erythrocyte damage in AIDS patients are mitigated.
The matsutake mushroom (*Tricholoma matsutake)* (*Accc51588*) is one of the most famous and precious edible fungi, and is often called "the king of the edible fungi". The matsutake mushroom is a delicacy rich in nutrients, delicious in taste and also fragrant. The matsutake mushroom (*Tricholoma matsutake)* has 17% protein content, 5.8% fat and 61.5% soluble non-nitrogen compounds, as well as being a rich source of vitamins B1, B2, and C, nicotinic acid, calcium, phosphorus, iron and others. It can improve patient health, benefit the intestines and stomach, relieve pain, regulate the flow of *qi* (for eliminating phlegm, expelling parasite etc.), and is effective in reducing glycuresis and in the inhibition of tumors. Some Japanese sources claim that the matsutake mushroom (*Tricholoma matsutake)* may reduce the rate of tumor growth by up to 90%. The mushroom (*Tricholoma matsutake)* also has a high market value. Its sale price in the international market is about 30∼50 thousand dollars per ton, which is higher than other fungi.
The common morel (*Morchella spp*.) (Accc51837), also known as morchella, is a hypha of *Morchella angusticeps Peck* (*Pezizales, Morohellaceae, Morchella*). It is widly distributed in Henan, Shanxi, Gansu, Qinghai, Xizang, Xinjiang, Sichuan, Shanxi, Jilin, Jiangsu, Yunnan, Hebei, Beijing and other provinces in China. It was first recorded in *Xinhua materia medica outline* that the effects of the common morel (*Morchella spp*.) calm the stomach, aid digestion, and regulate the flow of *qi* for the elimination of phlegm. It is mainly effective for treatment of the ailment known as "food stagnation" in TCM, abdominal fullness and distention, excess phlegm, the circulation of vital energy in the wrong direction, and dyspnea accompanied by a cough. It is sweet and mild, and is mainly effective on the spleen and stomach. The common morel (*Morchella spp*.) contains polysaccharides for inhibiting tumor growth as well as active anti-bacterial and anti-viral ingredients, and can strengthen the body's immunity. A high level of selenium is contained in *Morchella spp.,* which is an essential consistituent of glutathione peroxidase in human erythrocytes, and can transport large quantities of oxygen in order to inhibit cancer by inactiviating cancer cells and strengthening the anti-oxidization effect of vitamin E. According to *The Chinese Materia Medica,* the common morel (*Morchella spp*.) is sweet, cold and non-toxic. It is beneficial to the intestines and stomach, and reduces phlegm, regulates the flow of vital energy and removes obstructions therein. It is mainly effective for the treatment of indigestion, excessive phlegm and coughs. It is sweet and mild, beneficial to the intestines and stomach, aids digestion, reduces phlegm, regulates the flow of vital energy and removes obstructions thereto, "tonifies" the kidney *yin* in TCM, nourishes the brain and refreshes the patient. It has positive effects on weaknesses of the spleen and stomach, indigestion, excessive phlegm, shortness of breath, dizziness, and insomnia. The common morel (*Morchella spp*.) has a high level of organic germanium, which strengthens general health, prevents common colds and enhances the body's immunity. It also contains various essential mineral substances: the potassium and phosphorus content in every 100g of dry *Morchella spp.* is 7 times more and 4 times more, respectively, compared with that of the Chinese caterpillar fungus; the content of zinc in every 100g of dry *Morchella spp.* is 4.3 times that found in the shiitake mushroom, and 4 times that found in *Hericium erinaceus*; the content of iron in every 100g of dry *Morchella spp.* is 31 times that found in the shiitake mushroom, and 12 times that found in *Hericium erinaceus* (source: *Chinese Medicinal Mycology,* published in 1997). In this invention, the common morel (*Morchella spp*.) can be substituted with *Morchella conica Fr., Morchella conica Pers*., or *Morchella crassipes (Vent.) Pers.,* since these varieties of *Morchella spp.* are almost identical to the common morel (*Morchella spp*.) of the present invention in flavor, nature and effect.

Thus far, no-one else has investigated the effects of these two edible fungi on treating AIDS. In this invention, the beneficial effects on the stomach and intestines, the regulatory effect on the flow of vital energy and removal of obstructions thereto, the "tonifying" effect on the kidney *yin,* and the anti-viral effects of these two fungi are all exploited, and the effects of removing necrotic tissue and promoting granulation, as well as the anti-inflammatory effect of *Callicarpa bodinieri,* are all combined by co-fermentation. It may be assumed that the interaction and combination occurs during fermentation, with the consequence that the food is effective in relieving the symptom of "fear of chill" in AIDS patients, alleviating a series of complications due to erythrocyte damage, slowing down the course of the disease and improving the survival quality of AIDS patients.
During preparation, 60-140 hours of fermentation ensures complete fermentation and a high enough level of effective solid substances and polysaccharides, and results in the ideal therapeutic effect. The fermentation time also depends on the temperature outside the fermenting vessel, and on the nutrient and oxygen content of the medium during fermentation.
The food of this invention lists the co-fermentation products of *Callicarpa bodinieri, Morchella spp.* and *Tricholoma matsutake* as active ingredients. During preparation, an aqueous extract of *Callicarpa bodinieri* is used as a fermentation medium for the *Morchella spp.* and *Tricholoma matsutake.* In one embodiment, adjuvants for fermentation are added to the aqueous extract of *Callicarpa bodinieri.* Clinical data indicates that adjuvants added in a proper proportion do not affect the efficacy of the food of this invention.
The *Morchella spp.* and *Tricholoma matsutake* are mixed and injected into the aqueous extract of *Callicarpa bodinieri,* in which the fermentation is conducted. The fermentation liquor obtained is filtered and the filter residue is dried and prepared into granules weighing 0.34g. The granules may be used for oral administration at a dosage of no fewer than 6 pills and no more than 18 pills. Taking the food for 10 consecutive days will result in the AIDS patient efficiently achieving a better quality of life. The filtrate may also be sterilized and prepared as soup or subjected to concentration and crystallization to be prepared as powder. The soup or powder can be added to the patient's daily food as a nutritional supplement for AIDS patients. Taking 2.04∼6.12g of the soup or the powder (or food containing 2.04∼6.12g of the soup or the powder) every day can effectively strengthen the immune system of an AIDS patient, prevent complications, relieve symptoms and enhance organism activity.
Fermentation is conducted by adding the hyphae of maternal strains of *Morchella spp.* and *Tricholoma matsutake* to the aqueous extract of the *Callicarpa bodinieri* flower, stem, leaf or seed, and obtaining a fermentation liquor. The filter residue of the fermentation liquor is dried and prepared as granules; the filtrate was sterilized and then prepared as soup or subjected to concentration and crystallization to be prepared as powder. Said granules, soup or powder is taken by AIDS patients directly or added into their daily food as a nutritional supplement. Taking the food continuously can effectively strengthen the immune systems of AIDS patients, prevent complications, relieve symptoms and improve their organism's activity. Said nutritional food of this invention is an organic combination of various constituents, and its effects rely on the synergy of the various active ingredients. It can regulate the organism's function, effectively prevent erythrocyte decay, relieve and cure the symptom of "fear of chill" in AIDS patients, further improve general patient health by means of nutritional supplement, increase immunity, and accordingly inhibit complications, thus exerting the full effect of each ingredient in the food.
The nutritional food of this invention has been taken by 160 AIDS patients, and their symptoms of "fear of chill" were relieved gradually or even disappeared. Other accompanying symptoms to "fear of chill" were also relieved gradually, and no other new complications emerged. Combined with other AIDS medicine therapies, their quality of life was significantly improved, and the total rate of efficacy was above 96%.
Said granules, soup, or powder can be used as food additive to make a food for preventing AIDS. Eating a food having added said granules, soup, or powder in the long term can effectively prevent the symptom of "fear of chill" in AIDS patients.
The preparation of the food of this invention is simple and suitable for industrial, large-scale production. Said food has few side effects, a lasting therapeutic effect, and it's affordable for AIDS patients; it can prevent and relieve the symptom of "fear of chill" , enhance the organism' s immunity, improve physical markers of health, strengthen the organism' s activity and increase the quality of life of AIDS patients.

### BRIEF DESCRIPTION OF SELECTED ENMBODIMENTS

### Materials:

The root of the Purple Pearls beautyberry (*Callicarpa bodinieri)* was removed from its bark, and the plant "flesh" was obtained from the root; 1kg of the flesh was added to 20∼25Kg of water and boiled for 50 minutes to obtain 20∼25kg of aqueous extract No.1 of the Purple Pearls beautyberry (*Callicarpa bodinieri*).

Hyphae of maternal strains of *Morchella spp.* and *Tricholoma matsutake* were obtained by a spore separation method. Their hyphae, cultured on a slant or plate, were selected by microscopy and stored at 4°C for a period of less than 90 days.

The maternal strains of *Morchella spp.* and *Tricholoma matsutake* adopted in this invention were test-tube maternal strains (the test-tube being 18mm in diameter × 180mm in height) and purchased from Agricultural Culture Collection of China, the Chinese Academy of Agricultural Sciences. The *Tricholoma matsutake* has the deposit No.: Accc51588; the *Morchella spp.* has the deposit No.: Accc51837.
Said maternal strains are also stored at the applicant's laboratory, and will be available to the public for research use 20 years from the application filing date.

### Embodiment 1. Preparation of food No. 1

The aqueous extract No.1 of Purple Pearls beautyberry (*Callicarpa bodinieri*) was added to 20-25 volumes of drinking water to create a TCM liquid medium. Said TCM liquid medium was poured into a 200L fermenter, which is disclosed in the utility model patent "Tank cover of culture tank for liquor strain of edible fungi and culture tank" (patent No. CN03270560.3). 15% of the available space must be left empty in the fermenter and antifoaming agents or a defoamer must then be injected. (Said antifoaming agents or defoamer are conventional agents for edible fungi fermentation, and its selection as well as the selection on its consumption is conventional). When the flange cover was locked, the fermenter was moved into "the saturated steam pressureless boiler" (patent no. 200320126611.8) and left for 12 hours. When the fermenter was cooled to 26°C, the purchased test-tube maternal strains of *Morchella spp.* and *Tricholoma matsutake* were respectively injected into said fermenter (patent No. 03270560.3), and 50L of TCM liquid medium were injected with one tube of maternal strains of *Morchella spp.* and one tube of maternal strains of *Tricholoma matsutake* using the injector described in "Injection rod for edible fungi liquor and injector containing said injection rod" (patent no. 200320126960.X). After 60∼144 hours of fermentation, the fermenter was opened, and the resulting fermentation liquor was filtered. The filter residue was then dried to prepare granules, and the filtrate was inactivated and divided into two sections for preparing soup and powder respectively.

Granules gained by the above-mentioned process are suitable for oral administration to AIDS patients with dosage of no fewer than 6 pills and no more than 18 pills. Taking the granules once every day for 10 consecutive days will be effective to relieve or even cure the symptom of "fear of chill".

Said soup or powder (or other food forms that are prepared with said soup or powder by conventional processes, e.g. nutritional supplement powder, congee or gruel) are suitable for AIDS patients. Eating food containing 2.04∼6.12g of the soup or the powder every day for 10 consecutive days will relieve or even cure the symptom of "fear of chill", relieve other symptoms of complications correspondingly, and significantly improve the patient's quality of life.
Said granules, soup or powder can be used as nutritional supplement to prepare food for AIDS patients. Eating food supplemented with said granules, soup or powder can, in the long term, effectively prevent the symptom of "fear of chill" in AIDS patients.

The maternal strains of *Morchella spp* and *Tricholoma matsutake* used in this invention are purchased from Agricultural Culture Collection of China, Soil and Fertilizer Institute of the Chinese Academy of Agricultural Sciences. They are also stored at the applicant's laboratory, and will be available to the public for research in 20 years from the application filing date.

### Embodiment 2. Preparation of food No. 2

The aqueous extract No. 1 of *Callicarpa bodinieri* was added to 20-25 volumes of drinking water and other adjuvants (1 part pulse flour, 1 part corn flour, 2 parts white sugar, 0.1 part dipotassium hydrogen phosphate, 0.1 part potassium dihydrogen phosphate, 0.05 parts magnesium sulfate) amounting to 4∼5 times the weight of *Callicarpa bodinieri,* which are used to increase the density of the fermentation, yielding a TCM liquid medium. Said TCM liquid medium was poured into a 200L fermenter, as disclosed in the utility model patent "Tank cover of culture tank for liquor strain of edible fungi and culture tank" (patent No. CN03270560.3). 15% of the space in the fermenter was left empty and moderate antifoaming agents or a defoamer (said antifoaming agents or defoamer are conventional agents for edible fungi fermentation, and their selection as well as the selection on their consumption is conventional) were injected. When the flange cover is locked, said fermenter is moved into "the saturated steam pressureless boiler" (patent no. 200320126611.8) and left for 12 hours for inactivation. When the fermenter was cooled to 26°C, the purchased tube maternal strains of *Morchella spp.* and *Tricholoma matsutake* were respectively injected into said fermenter (patent No. 03270560.3), and 50L of TCM liquid medium were injected with one tube of maternal strains of *Morchella spp.* and one tube of maternal strains of *Tricholoma matsutake* by means of the injector recorded in "injection rod for edible fungi liquor and injector containing said injection rod" (patent no. 200320126960.X). After 60∼144 hours of fermentation, the fermenter was opened and the resulting fermentation liquor was filtered. The filter residue was then dried to prepare granules, and the filtrate was inactivated and divided into two sections for preparing soup and powder respectively.

Granules gained by the above-mentioned process are suitable for oral administration to AIDS patients at a dosage of no fewer than 6 pills and no more than 18 pills, and taking the granules for 10 consecutive days will be effective to relieve or even cure the symptom of "fear of chill". Eating food containing 2.04∼6.12g of said soup or powder once every day for 10 consecutive days can relieve the symptom gradually and enhance quality of life significantly.

Said granules, soup, or powder can be used as nutritional supplement to prepare food for AIDS patients. Eating food supplemented with said granules, soup, or powder can effectively improve immunity in the long term.

The maternal strains of *Morchella spp.* and *Tricholoma matsutake* used in this invention are purchased from Agricultural Culture Collection of China, Soil and Fertilizer Institute of the Chinese Academy of Agricultural Sciences. They are also stored at the applicant's laboratory, and will be available to the public for research in 20 years from the application filing date.

### Example 1. Application effect of granules of food obtained in embodiment 1

In this invention, standard of quality of life refers to the quality of life questionnaire for Chinese cancer patients (QLQ-CCC) compiled by Professor Yan SUN of department of internal medicine, the Affiliated Tumor Hospital of China Academy of Medical Science, which intended is for assessing the quality of life of AIDS patients. There are 12 criteria including: mental state, appetite, sleep, symptoms, therapy, understanding & support, daily life, and others set forth in QLQ-CCC. In each item, there are 5 grades from 1 to 5 points, where a high score means a better quality of life. A full score is 60 points, and the lowest score possible is 12 points. If the score is lower than 20 points, quality of life is considered "very poor"; at 21-30 points, quality of life is considered "poor"; at 31-40 points, quality of life is considered "average"; at 41-50 points, quality of life is assessed as "good"; and at 51-60 points the assessment is "very good". AIDS patients fill in QLQ-CCC once before and once after eating the food of this invention.

There were 100 volunteers with AIDS, whose blood examination result was HIV positive (+). Before this test, all of them had one or more of the following symptoms: fever, shivers, joint pain, muscle pain, vomiting, diarrhea, laryngalgia with persistent fever, night sweat, enervation, systemic superficial lymphadenopathy, body weight loss over 10% in 3 months (and up to 40%), becoming visibly thin, appetite decreasing, apocleisis, sicchasia, hematochezia, dizziness, headache, lags in response, hypophrenia, insanity, convulsions, hemiplegic paralysis, dementia, diffuse papules, acute posterior ganglionitis, inflammation and decay in the oral cavity and pharyngeal mucosa. Taking said granules of food No.1 once every day for 10 consecutive days, 96 volunteers indicated that their pain-related and "fear of chill" symptoms were relieved, and even disappeared. Other complications were relieved significantly and even disappeared. None of the patients developed new complications, and their mental states became better. Furthermore, according to the data (regarding the distribution of quality of life scores before and after taking granules of food no.1), the total effective rate of treatment was as high as 96%. The data for distribution of quality of life score before and after eating granules of food no.1 is shown in table 1.

**Table 1: the data distribution of life quality score before and after eating said granules of food obtained in embodiment 1**

| Number of AIDS patients | Time | life quality score (point) | | | | |
|---|---|---|---|---|---|---|
| | | <20 | 21-30 | 31-40 | 41-50 | 51-60 |
| 100 | Before eating granules of food no.1 | 4 | 44 | 40 | 10 | 2 |
| | After eating granules of food no. 1 | 0 | 4 | 36 | 48 | 12 |

### Typical case:

Name: XXX; sex: X; born in XX; address: XX; profession: X
Blood examination result: HIV positive (+)
Symptoms: apocleisis, fatigue and lack of strength, diarrhea, dizziness, headache, lags in response, hypophrenia, insanity, systemic pruritus. XXX took granules of food No.1. During the period whilst they were taking the food, there were no symptoms of gastrointestinal upset, diarrhea and etc. After taking granules of food No.1, there was no abnormality in hepatorenal function indicators, and no obvious change in the patient's hemogram; the "fear of chill" symptom was relieved, and even disappeared; the body weight as well as appetite of the patient was improved, and their mental state became better; colleagues and friends of the patient deemed that the patient was significantly improved. With the active coordination of medicine therapy, the patient's life quality was obviously improved.

### Example 2. Application effect of soup of food obtained in embodiment 1

There were 100 volunteers with AIDS, whose blood examination results were HIV positive (+). Before taking the food, all of them had one or more of the following symptoms: fever, shivers, joint pain, muscle pain, vomiting, diarrhea, laryngalgia with persistent fever, night sweat, enervation, systemic superficial lymphadenopathy, body weight loss over 10% in 3 months (and up to 40%), becoming visibly thin, appetite decreasing, apocleisis, sicchasia, hematochezia, dizziness, headache, dulled reflexes, hypophrenia, insanity, convulsions, hemiplegic paralysis, dementia, diffuse papules, acute posterior ganglionitis, inflammation and decay in the oral cavity and pharyngeal mucosa. Taking said granules of food No.1 for 10 consecutive days, 97 volunteers reported that their pain-related and "fear of chill" symptoms were relieved and even disappeared, and complications were clearly relieved and even disappeared. No new complications arose in any of the patients and their mental states became better. Furthermore, according to data (regarding the distribution of quality of life scores before and after eating the soup of food No.1), the total effective rate of treatment was as high as 97%. The distribution of quality of life score data before and after eating the soup of food No.1 is shown below in table 1.

**Table 1: the data distribution of life quality score before and after eating said soup of food obtained in embodiment 1**

| Number of AIDS patients | Time | life quality score (point) | | | | |
|---|---|---|---|---|---|---|
| | | <20 | 21-30 | 31-40 | 41-50 | 51-60 |
| 100 | Before eating soup of food no.1 | 4 | 44 | 40 | 10 | 2 |
| | After eating soup of food no.1 | 0 | 3 | 36 | 49 | 12 |

### Typical case:

Name: XXX ; sex: X ; born in XX ; address: XX ; profession:
Blood examination result: HIV positive (+)
Symptoms: apocleisis, skin being easy to be damaged with haemophilia, fatigue and lack of strength, diarrhoea, dizziness, headache, lags in response, hypophrenia, insanity, systemic pruritus. XXX took soup of food No. 1. During the period of consumption, there were no symptoms of gastrointestinal upset, or diarrhea etc. After taking soup of food No. 1, there was no abnormality in hepatorenal function indicators and no obvious change in the patient's hemogram. The symptom of "fear of chill" was relieved and even disappeared. The body weight as well as the appetite of the patient increased, and their mental state became better; colleagues and friends of the patient admitted the patient was significantly improved. In careful conjunction with medicine therapy, the patient's life quality was obviously improved.

### Example 3. Application effect of powder obtained in embodiment 1

There were 100 volunteers with AIDS, whose blood examination results were HIV positive (+). Before taking the food, they all had one or more of the following symptoms: fever, shivers, joint pain, muscle pain, vomiting, diarrhea, laryngalgia with persistent fever, night sweat, enervation, systemic superficial lymphadenopathy, body weight loss over 10% in 3 months (up to 40% at most), becoming visibly thin, appetite decreasing, apocleisis, sicchasia, hematochezia, dizziness, headache, lags in response, hypophrenia, insanity, convulsions, hemiplegic paralysis, dementia, diffuse papules, acute posterior ganglionitis, inflammation and decay of the oral cavity and pharyngeal mucosa. Taking said powder of food No.1 once every day for 10 consecutive days 97 volunteers indicated that their pain-related and "fear of chill" symptoms were relieved and even disappeared. Complications were clearly relieved and even disappeared, there were no new complications raised in any of the patients, and their mental states became better. Furthermore, according to the data regarding distribution of life quality score before and after eating powder of food No.1, the total effective rate of treatment was as high as 97%. The data regarding distribution of life quality score before and after eating powder of food No.1 is shown in table 1.

**Table 1: the data distribution of life quality score before and after eating said powder of food obtained in embodiment. 1**

| Number of AIDS patients | Time | life quality score (point) | | | | |
|---|---|---|---|---|---|---|
| | | <20 | 21-30 | 31-40 | 41-50 | 51-60 |
| 100 | Before eating powder of food no.1 | 4 | 44 | 40 | 10 | 2 |
| | After eating powder of food no.1 | 0 | 3 | 36 | 48 | 12 |

### Typical case:

Name:XXX ; sex: X ; born in XX ; address: XX ; profession:
Blood examination result: HIV positive (+)
Symptoms: persistent fever, apocleisis, fatigue and lack of strength, enervation, night sweat, diarrhoea, dizziness, headache, lags in response, hypophrenia, insanity, systemic pruritus. XXX ingested a powder of food no. 1. During the period of consumption, there were no symptoms of gastrointestinal upset, or diarrhea etc. After consuming a soup with food No. 1, there was no abnormality in hepatorenal function indicators and no obvious change in the patient's hemogram; the symptom of "fear of chill" was relieved and even disappeared; the patient's body weight as well as appetite increased, and their mental state became better; colleagues and friends of the patient admitted that the patient was significantly improved. In careful coordination with medicine therapy, the patient's life quality was clearly improved.

### Example 4. Application effect of granules obtained in embodiment 2

There were 60 volunteers with AIDS, whose blood examination result was HIV positive (+). Before this test they all had one or more of following symptoms: fever, shivering, joint pain, muscle pain, vomiting, diarrhea, laryngalgia with persistent fever, night sweat, enervation, systemic superficial lymphadenopathy, body weight loss over 10% in 3 months (and even up to 40%), becoming visibly thin, appetite decreasing, apocleisis, sicchasia, hematochezia, dizziness, headache, lags in response, hypophrenia, insanity, convulsions, hemiplegic paralysis, dementia, diffuse papules, acute posterior ganglionitis, inflammation and decay to the oral cavity and pharyngeal mucosa. Taking said granules of food No.2 once every day for 10 consecutive days, 58 volunteers reported that their pain-related and "fear of chill" symptoms were relieved and even disappeared. Other complications were clearly relieved and even disappeared. None of the patients suffered any new complications, and their mental states became better. Furthermore, according to the data collected for distribution of life quality score before and after consuming granules of food No.2, the total efficacy rate of the treatment was as high as 96.67%. The data regarding distribution of life quality score before and after eating granules of food No. 2 is shown in Table 2.

**Table 2: the data distribution of life quality score before and after eating said granules of food obtained in embodiment 2**

| Number of AIDS patients | Time | life quality score (point) | | | | |
|---|---|---|---|---|---|---|
| | | <20 | 21-30 | 31-40 | 41-50 | 51-60 |
| 60 | Before eating granules of food no.2 | 2 | 22 | 20 | 14 | 2 |
| | After eating granules of food no.2 | 0 | 2 | 13 | 37 | 8 |

### Typical case:

XXX sex: X born in XX address: XX profession:
Blood examination result: HIV positive (+)
Symptoms: skin easily damaged on account of haemophilia, persistent fever, enervation, night sweat, decrease in appetite, flusteredness, dizziness, fatigue and lack of strength, drowsiness, lags in response, hypophrenia. XXX took granules of food No. 2. During the period of consumption, there was no symptom of gastrointestinal upset, diarrhea and etc. After taking granules of food No.2, the symptom "fear of chill" disappeared gradually; bodily strength was significantly recovered; no new complications arose; body weight and immunity were enhanced, and the patient could actively accept AIDS medicine therapy.

### Example 5. Application effect of soup obtained in embodiment 2

There were 60 volunteers with AIDS, whose blood examination results were HIV positive (+). Before this test they all had one or more of following symptoms: fever, shivers, joint pain, muscle pain, vomiting, diarrhea, laryngalgia with persistent fever, night sweats, enervation, systemic superficial lymphadenopathy, body weight loss over 10% in 3 months (and up to 40%), becoming visibly thin, appetite decreasing, apocleisis, sicchasia, hematochezia, dizziness, headache, lags in response, hypophrenia, insanity, convulsions, hemiplegic paralysis, dementia, diffuse papules, acute posterior ganglionitis, inflammation and decay of the oral cavity and pharyngeal mucosa. Taking said soup of food No.2 once every day for 10 consecutive days, 58 volunteers reported that their symptoms relating to pain and "fear of chill" were relieved and even disappeared. Other complications were relieved obviously and even disappeared, no new complications arose in any of the patients, and their mental states became better. Furthermore, according to the data regarding distribution of life quality score before and after taking soup of food No.2, the total rate of efficacy was as high as 96.67%. The data concerning distribution of life quality score before and after eating soup of food No.2 is shown in table 2.

**Table 2: the data distribution of life quality score before and after eating said soup of food obtained in embodiment 2**

| Number of AIDS patients | Time | life quality score (point) | | | | |
|---|---|---|---|---|---|---|
| | | <20 | 21-30 | 31-40 | 41-50 | 51-60 |
| 60 | Before eating soup of food no.2 | 2 | 22 | 20 | 14 | 2 |
| | After eating soup of food no.2 | 0 | 1 | 13 | 38 | 8 |

### Typical case:

XXX sex: X born in XX address: XX profession:
Blood examination result: HIV positive (+)
Symptoms: diarrhea, persistent fever, enervation, night sweats, skin easily damaged on account of haemophilia, appetite decreasing, flusteredness, dizziness, fatigue and lack of strength, drowsiness, lags in response, hypophrenia. XXX took a soup of food No. 2. During the period of consumption, there were no symptoms of gastrointestinal upset, diarrhea etc. After taking soup of food No. 2, the symptom "fear of chill" disappeared gradually; no new complications arose; the patients' physical strength was recovered; their body weight increased; immunity was enhanced, and they were able to actively receive AIDS medicine therapy.

### Example 6. Application effect of powder obtained in embodiment 2

There were 80 volunteers with AIDS, whose blood examination results were HIV positive (+). Before this test they all had one or more of the following symptoms: fever, shivers, joint pain, muscle pain, vomiting, diarrhea, laryngalgia with persistent fever, night sweat, enervation, systemic superficial lymphadenopathy, body weight loss over 10% in 3 months (and up to 40%), becoming visibly thin, appetite decreasing, apocleisis, sicchasia, hematochezia, dizziness, headache, lags in response, hypophrenia, insanity, convulsions, hemiplegic paralysis, dementia, diffuse papules, acute posterior ganglionitis, inflammation and decay of the oral cavity and pharyngeal mucosa. Taking said powder of food No.2 once every day for 10 consecutive days, 77 volunteers reported that their symptoms related to pain and "fear of chill" were relieved and even disappeared; other complications were clearly relieved and even disappeared, no new complications arose in any patients, and their mental states became better. Furthermore, according to the data regarding the distribution of life quality scores before and after taking the powder of food No.2, the total efficacy rate of the treatment was as high as 96.25%. The data regarding the distribution of life quality scores before and after eating powder of food No.2 is shown in table 2.

**Table 2: the data distribution of life quality score before and after eating said powder of food No.2**

| Number of AIDS patients | Time | life quality score (point) | | | | |
|---|---|---|---|---|---|---|
| | | <20 | 21-30 | 31-40 | 41-50 | 51-60 |
| 60 | Before eating powder of food no.2 | 6 | 26 | 24 | 18 | 6 |
| | After eating powder of food no.2 | 0 | 3 | 18 | 44 | 15 |

### Typical case:

XXX sex: X born in XX address: XX profession:
Blood examination result: HIV positive (+)
Symptoms: dizziness, systemic pruritus, skin being easy to be damaged with haemophilia, persistent fever, enervation, night sweat, appetite decreasing, insanity, flusteredness, headache, fatigue and lack of strength, drowsiness, lags in response, hypophrenia. During consumption of the food powder obtained in embodiment 1, there were no symptoms of gastrointestinal upset, diarrhea or other complications. After taking of the powder, the symptom of "fear of chill" disappeared gradually; their physical strength was recovered; body weight as well as immunity of the patient increased and they were able to actively accept AIDS medicine therapy.

In this invention, 60 AIDS patients were invited as volunteers to eat the food containing 2.04∼6.12g granules, soup or powder of food no. 1 and food no. 2 respectively for 10 consecutive days, and the symptom of "fear of chill" in more than 96% of the patients was gradually relieved or even disappeared; other symptoms were relieved correspondingly. The patients' quality of life was significantly improved.

In addition, the inventor invited 120 volunteers who have just been diagnosed with HIV (HIV positive) to take the food of the invention. Said 120 volunteers were divided into 12 groups with 10 persons in each group, and they took 2.04∼6.12g granules, soup or powder of food No. 1 and food No. 2; or food containing 2.04∼6.12g granules, soup or powder of food No. 1 and food No. 2 respectively for half a year. They were subjected to an HIV test and an complications test after the research period of half a year ended, and the results showed that the onset of complications occurred later in all volunteers' body as compared with ordinary AIDS patients. The food was 100% effective in slowing down the disease course of AIDS. These results demonstrate that eating said granules, soup or powder of this invention in the long term or eating food containing said granules, soup or powder of this invention in the long term can effectively prevent AIDS.

With regard to AIDS patients who eat said food for nutritional therapy of AIDS according to this invention, the above examples show that their systemic nutritional states are improved; the decay of erythrocytes is significantly inhibited; the symptom of "fear of chill" was relieved and cured; their body weights are no longer basically decreased; and opportunistic infections were controlled. Eating the food of this invention can establish a base for further medical therapy, strengthen the confidence of AIDS patients and significantly improve their life quality.

## Claims

1. A food suitable for nutritional therapy of AIDS, wherein the food is prepared by fermentation of hyphae of a maternal strain of common morel and hyphae of a maternal strain of matsutake mushroom in traditional Chinese medicine liquid medium, and said traditional Chinese medicine liquid medium is an aqueous extract of raw material of *Callicarpa bodinieri,* wherein the weight ratio between the original raw material and the final aqueous extract of the raw material is 4∼5%, wherein said common morel is *Morchella spp.* Accc51837, and wherein said matsutake mushroom is *Tricholoma matsutake* Accc51588.

2. The food claim 1, wherein said traditional Chinese medicine medium also comprises an adjuvant, and the preparation of the food includes diluting said aqueous extract by adding 20∼25 volumes of water, then adding adjuvant into the aqueous extract, wherein the added adjuvant has 4∼5 times the weight of the raw material; then conducting elixation, finally taking the juice and removing the slag, wherein the components of said adjuvant by part of weight are as follows: 1 part of pulse flour, 1 part of corn flour, 2 parts of white sugar, 0.1 parts of dipotassium hydrogen phosphate, 0.1 parts of potassium dihydrogen phosphate, 0.05 parts of magnesium sulfate.

3. The food according to claim 1 or 2, wherein said aqueous extract is extracted from the root of *Callicarpa bodinieri.*

4. The food according to claim 3, wherein the form of the food is granules, soup or powder.

5. Preparation method of a food for nutritional therapy of AIDS, comprising the steps of
injecting hyphae of maternal strains of common morel and matsutake mushroom into traditional Chinese medicine liquid medium and
conducting fermentation, wherein said traditional Chinese medicine liquid medium is an aqueous extract of raw material of *Callicarpa bodinieri,* wherein the weight ratio between the original raw material and the aqueous extract of the raw material is 4∼5%, wherein said common morel is *Morchella spp.* Accc51837, and wherein said matsutake mushroom is *Tricholoma matsutake* Accc51588.

6. The method of claim 5, wherein said fermentation comprises:
pouring said traditional Chinese medicine liquid medium into a fermenter,
inactivation by water steam under ordinary pressure,
cooling to 26±2°C; and then
injecting common morel and matsutake mushroom into said fermenter, and fermenting for 60-144 hours.

7. The method of claim 6, wherein said aqueous extract is extracted from the root of *Callicarpa bodinieri.*

8. Preparation method of a food for nutritional therapy of AIDS, comprising the steps of:
injecting hyphae of maternal strains of common morel and matsutake mushroom in traditional Chinese medicine liquid medium and
conducting a fermentation, wherein said common morel is *Morchella spp* Accc51837, said matsutake mushroom is *Tricholoma matsutake* Accc51588; said traditional Chinese medicine liquid medium is an aqueous extract of the raw material of *Callicarpa bodinieri,* and wherein the weight ratio between the original raw material and the final aqueous extract of the raw material is 4-5%,
diluting said aqueous extract by adding 20∼25 volumes of water,
then adding adjuvant into the aqueous extract, wherein the added adjuvant has 4-5 times the weight of the raw material;
then conducting elixation, finally taking the juice and removing the slag,
wherein the components of said adjuvant by part of weight are as follows: 1 part of pulse flour, 1 part of corn flour, 2 parts of white sugar, 0.1 parts of dipotassium hydrogen phosphate, 0.1 parts of potassium dihydrogen phosphate, 0.05 parts of magnesium sulfate.

9. The method of claim 8, wherein said fermentation comprises:
pouring said traditional Chinese medicine liquid medium into a fermenter,
inactivation by water steam under ordinary pressure,
cooling to 26±2°C; and then
injecting said common morel and said matsutake mushroom into said fermenter, and fermenting for 60-144 hours.

10. The method of claim 9, wherein, said aqueous extract is extracted from the root of *Callicarpa bodinieri.*

## Patentansprüche

1. Ein Nahrungsmittel, geeignet zur Ernährungstherapie von AIDS, wobei das Nahrungsmittel hergestellt wird durch die Fermentation von Hyphen eines Elternstamms einer Speisemorchel und Hyphen eines Elternstamms eines Matsutake Pilzes in einem flüssigen Medium der traditionellen Chinesischen Medizin, und besagtes flüssiges Medium der traditionellen Chinesischen Medizin ist ein wässriger Extrakt von Rohmaterial von *Callicarpa bodinieri,* wobei das Gewichtsverhältnis von dem ursprünglichen Rohmaterial und dem endgültigen wässrigen Extrakt des Rohmaterials 4-5% ist, wobei die Speisemorchel *Morchella spp.* Accc51837, und wobei der besagte Matsutake Pilz *Tricholoma matsutake* Accc51588 ist.

2. Das Nahrungsmittel gemäß Anspruch 1, wobei das besagte Medium der traditionellen Chinesischen Medizin ferner ein Adjuvant umfasst, und die Herstellung des Nahrungsmittels die Verdünnung des genannten wässrigen Extrakts durch 20-25 Volumen Wasser, anschließende Zugabe von Adjuvant, wobei das zugegebene Adjuvant 4-5 Mal das Gewicht des Rohmaterials besitzt; anschließendes Elixieren und abschließendes Gewinnen des Safts und Entfernen des Rückstands einschließt, wobei die Bestandteile des genannten Adjuvants in Gewichtsanteilen die folgenden sind: 1 Teil Hülsenfruchtmehl, 1 Teil Maismehl, 2 Teile Weißzucker, 0,1 Teile Dikaliumhydrogenphosphat, 0,1 Teile Kaliumdihydrogenphosphat, 0,05 Teile Magnesiumsulfat.

3. Das Nahrungsmittel gemäß Anspruch 1 oder 2, wobei der besagte wässrige Extrakt von den Wurzeln des *Callicarpa bodinieri* extrahiert ist.

4. Das Nahrungsmittel gemäß Anspruch 3, wobei die Form des Nahrungsmittels Granulat, Suppe, oder Pulver ist.

5. Herstellungsverfahren für ein Nahrungsmittel zur Ernährungstherapie von AIDS, umfassend die Schritte:
Injizieren von Hyphen von Elternstämmen einer Speisemorchel eines Matsutake Pilzes in ein flüssiges Medium der traditionellen Chinesischen Medizin und
Durchführen einer Fermentation, wobei das besagte flüssige Medium der traditionellen Chinesischen Medizin ein wässriger Extrakt von Rohmaterial von *Callicarpa bodinieri* ist, wobei das Gewichtsverhältnis von dem ursprünglichen Rohmaterial und dem wässrigen Extrakt des Rohmaterials 4-5% ist, wobei die Speisemorchel *Morchella spp.* Accc51837 ist, und wobei der besagte Matsutake Pilz *Tricholoma matsutake* Accc51588 ist.

6. Das Verfahren gemäß Anspruch 5, wobei die Fermentation umfasst:
Einfüllen des besagten flüssigen Mediums der traditionellen Chinesischen Medizin in einen Fermenter,
Inaktivierung durch Wasserdampf bei Umgebungsdruck,
Abkühlen auf 26±2 °C; und anschließend
Injizieren von Speisemorchel und Matsutake Pilz in den Fermenter, und Fermentation für 60-144 Stunden.

7. Das Verfahren gemäß Anspruch 6, wobei der wässrige Extrakt aus der Wurzel von *Callicarpa bodinieri* extrahiert ist.

8. Herstellung eines Nahrungsmittels zur Ernährungstherapie von AIDS, umfassend die Schritte:
Injizieren von Hyphen von Elternstämmen einer Speisemorchel eines Matsutake Pilzes in ein flüssiges Medium der traditionellen Chinesischen Medizin und
Durchführen einer Fermentation, wobei die Speisemorchel *Morchella spp.* Accc51837 ist, und wobei der besagte Matsutake Pilz *Tricholoma matsutake* Accc51588 ist; das besagte flüssige Medium der traditionellen Chinesischen Medizin ein wässriger Extrakt von Rohmaterial von *Callicarpa bodinieri* ist, und wobei das Gewichtsverhältnis von dem ursprünglichen Rohmaterial und dem endgültigen wässrigen Extrakt des Rohmaterials 4-5% ist,
Verdünnen des besagten wässrigen Extraktes durch Zugabe von 20-25 Volumen Wasser,
anschließend Zugabe von Adjuvant in den wässrigen Extrakt, wobei das zugegebene Adjuvant das 4-5 fache Gewicht des Rohmaterials hat;
anschließend Elixieren und abschließendes Gewinnen des Safts und Entfernen des Rückstands,
wobei die Bestandteile des genannten Adjuvants in Gewichtsanteilen die folgenden sind: 1 Teil Hülsenfruchtmehl, 1 Teil Maismehl, 2 Teile Weißzucker, 0,1 Teile Dikaliumhydrogenphosphat, 0,1 Teile Kaliumdihydrogenphosphat, 0,05 Teile Magnesiumsulfat.

9. Das Verfahren gemäß Anspruch 8, wobei die besagte Fermentation umfasst:
Einfüllen des besagten flüssigen Mediums der traditionellen Chinesischen Medizin in einen Fermenter,
Inaktivierung durch Wasserdampf bei Umgebungsdruck,
Abkühlen auf 26±2 °C; und anschließend
Injizieren von Speisemorchel und Matsutake Pilz in den Fermenter, und Fermentation für 60-144 Stunden.

10. Das Verfahren gemäß Anspruch 9, wobei der wässrige Extrakt aus der Wurzel von *Callicarpa bodinieri* extrahiert ist.

## Revendications

1. Aliment convenant à la thérapie nutritionnelle du SIDA, l'aliment étant préparé par la fermentation d'hyphes d'une souche maternelle de morille commune et d'hyphes d'une souche maternelle d'un champignon matsutake dans du milieu liquide de médecine traditionnelle chinoise, et ledit milieu liquide de médecine traditionnelle chinoise étant un extrait aqueux d'une matière première de *Callicarpa bodinieri,* le rapport en poids entre la matière première d'origine et l'extrait aqueux final de la matière première étant de 4 ∼ 5 %, ladite morille commune étant *Morchella spp.* Accc51837, et ledit champignon matsutake étant *Tricholoma matsutake* Accc51588.

2. Aliment selon la revendication 1, ledit milieu de médecine traditionnelle chinoise comprenant également un adjuvant, et la préparation de l'aliment comprenant la dilution dudit extrait aqueux par l'addition de 20 à 25 volumes d'eau, puis l'addition d'adjuvant dans l'extrait aqueux, l'adjuvant ajouté ayant de 4 à 5 fois le poids de la matière première ; puis la conduite d'une élixation, en prélevant finalement le jus et en retirant le résidu solide, les constituants dudit adjuvant en partie en poids étant les suivants : 1 partie de farine de pois, 1 partie de farine de maïs, 2 parties de sucre blanc, 0,1 partie d'hydrogénophosphate dipotassique, 0,1 partie de dihydrogénophosphate de potassium, 0,05 partie de sulfate de magnésium.

3. Aliment selon la revendication 1 ou 2, ledit extrait aqueux étant extrait de la racine de *Callicarpa bodinieri.*

4. Aliment selon la revendication 3, la forme de l'aliment étant des granulés, de la soupe ou une poudre.

5. Procédé de préparation d'un aliment pour la thérapie nutritionnelle du SIDA, comprenant les étapes
d'injection d'hyphes de souches maternelles de morille commune et de champignon matsutake dans un milieu liquide de médecine traditionnelle chinoise et
d'exécution d'une fermentation, ledit milieu liquide de médecine traditionnelle chinoise étant un extrait aqueux d'une matière première de *Callicarpa bodinieri,* le rapport en poids entre la matière première d'origine et l'extrait aqueux de la matière première étant de 4 ∼ 5 %, ladite morille commune étant *Morchella spp.* Accc51837, et ledit champignon matsutake étant *Tricholoma matsutake* Accc51588.

6. Procédé selon la revendication 5, ladite fermentation comprenant :
le fait de verser ledit milieu liquide de médecine traditionnelle chinoise dans un dispositif de fermentation,
l'inactivation à la vapeur d'eau sous pression ordinaire,
le refroidissement à 26 ± 2°C ; et ensuite
l'injection de la morille commune et du champignon matsutake dans ledit dispositif de fermentation, et la fermentation durant 60 à 144 heures.

7. Procédé selon la revendication 6, ledit extrait aqueux étant extrait de la racine de *Callicarpa bodinieri.*

8. Procédé de préparation d'un aliment pour la thérapie nutritionnelle du SIDA, comprenant les étapes :
d'injection d'hyphes de souches maternelles de morille commune et de champignon matsutake dans un milieu liquide de médecine traditionnelle chinoise et
d'exécution d'une fermentation, ladite morille commune étant *Morchella spp* Accc51837, ledit champignon matsutake étant *Tricholoma matsutake* Accc51588 ; ledit milieu liquide de médecine traditionnelle chinoise étant un extrait aqueux de la matière première de *Callicarpa bodinieri,* et le rapport en poids entre la matière première d'origine et l'extrait aqueux final de la matière première étant de 4 ∼ 5 %,
de dilution dudit extrait aqueux en ajoutant de 20 à 25 volumes d'eau,
puis d'addition d'un adjuvant dans l'extrait aqueux, l'adjuvant ajouté ayant de 4 à 5 fois le poids de la matière première ;
puis de conduite d'une élixation, en prélevant finalement le jus et en retirant le résidu solide,
les constituants dudit adjuvant en partie en poids étant les suivants : 1 partie de farine de pois, 1 partie de farine de maïs, 2 parties de sucre blanc, 0,1 partie d'hydrogénophosphate dipotassique, 0,1 partie de dihydrogénophosphate de potassium, 0,05 partie de sulfate de magnésium.

9. Procédé selon la revendication 8, ladite fermentation comprenant :
le fait de verser ledit milieu liquide de médecine traditionnelle chinoise dans un dispositif de fermentation,
l'inactivation à la vapeur d'eau sous pression ordinaire,
le refroidissement à 26 ± 2°C ; et ensuite
l'injection de ladite morille commune et dudit champignon matsutake dans ledit dispositif de fermentation, et la fermentation durant 60 à 144 heures.

10. Procédé selon la revendication 9, ledit extrait aqueux étant extrait de la racine de *Callicarpa bodinieri.*
